(19) Europäisches Patentamt
European Patent Office
Office européen des brevets

(11) Numéro de publication: **0 350 395 A1**

(12) **DEMANDE DE BREVET EUROPEEN**

(21) Numéro de dépôt: **89401920.7**

(22) Date de dépôt: **05.07.89**

(51) Int. Cl.$^5$: **C 07 C 45/29**
C 07 C 47/575, C 07 C 46/02,
C 07 C 50/28

(30) Priorité: **06.07.88 FR 8809169**

(43) Date de publication de la demande:
**10.01.90 Bulletin 90/02**

(84) Etats contractants désignés:
**AT BE CH DE ES FR GB GR IT LI LU NL SE**

(71) Demandeur: **SOCIETE NATIONALE ELF AQUITAINE**
**Tour Elf 2 Place de la Coupole La Défense 6**
**F-92400 Courbevoie (FR)**

(72) Inventeur: **Meunier, Bernard**
**Résidence les Ormes - Bât C3**
**F-31320 Castanet (FR)**

**Labat, Gilles**
**45, rue George Sand**
**F-31400 Toulouse (FR)**

**Seris, Jean-Louis**
**Lotissement Parenche No 15 Chemin Vignats**
**F-64110 Jurançon (FR)**

(74) Mandataire: **Ohresser, François**
**Société Nationale Elf Aquitaine Division Proprieté Industrielle Tour Elf**
**F-92078 Paris La Défense Cédex 45 (FR)**

(54) **Procédé d'oxydation par catalyse biomimétique d'alcools benzyliques et de composés apparentés.**

(57) L'invention a pour objet un procédé d'oxydation par catalyse biomimétique d'alcools benzyliques et de composés apparentés.

Le procédé de l'invention est caractérisé par l'utilisation d'un catalyseur constitué d'une métalloporphyrine dont le métal est le fer ou le manganèse, en présence d'un agent donneur d'oxygène consistant en un dérivé hydrosoluble d'un peracide organique ou minéral.

Le procédé de l'invention pourra notamment être utilisé pour la délignification de la pulpe de bois en vue d'obtenir des composés aromatiques.

EP 0 350 395 A1

## Description

# PROCEDE D'OXYDATION PAR CATALYSE BIOMIMETIQUE D'ALCOOLS BENZYLIQUES ET DE COMPOSES APPARENTES

La présente invention a pour objet un procédé d'oxydation par catalyse biomimétique d'alcools benzyliques et de composés apparentés.

Dans la suite, on désignera par composés apparentés des alcools benzyliques les composés comportant au moins un motif de formule :

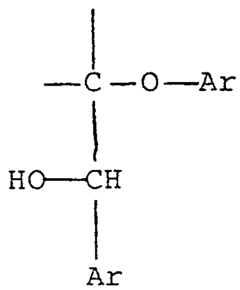

dans laquelle Ar représente un noyau aromatique substitué ou non.

Ces composés apparentés se retrouvent dans la lignine, polymére naturel, qui à côté de la cellulose et de l'hémi-cellulose, est un composant essentiel du bois. Il s'agit d'un polymère de nature aromatique à motifs type phénylpropane, le noyau aromatique portant un, deux ou trois radicaux hydroxy ou méthoxy.

La lignine, disponible en très grandes quantités, a longtemps été considérée comme un déchet sans utilité industrielle. Elle a essentiellement été utilisée, à côté de quelques applications spécifiques marginales, comme substitut de combustibles. La source principale de lignine est l'industrie papetière qui est amenée à délignifier la pulpe de bois afin de purifier les celluloses constituant la base de la pâte à papier.

Les problèmes d'environnement posés par les procédés actuellement mis en oeuvre et la possibilité d'utiliser la lignine comme source renouvelable de molécules aromatiques sont à l'origine depuis peu de temps d'importants efforts de recherche sur la dégradation oxydative de la lignine par voie chimique et/ou enzymatique.

L'approche oxydation par voie enzymatique a fait l'objet de nombreux travaux depuis la purification et la caractérisation d'enzymes extraites de champignons impliqués dans les processus de pourrissement du bois. Une enzyme, la ligninase a été isolée d'un champignon, Phanerochaete Chrysosporium. Il s'agit en fait d'une peroxydase possédant une protoporphyrine de fer (III) comme groupe prosthétique. Les études de son mode d'action indiquent que l'étape essentielle est la formation de radicaux-cations selon un processus de type peroxydase plutôt que de type mono-oxygénase comme on avait pu le penser initialement. A côté de cette enzyme à fer, une autre peroxydase extracellulaire à manganèse a été également isolée.

Ces résultats ont amené les chercheurs à utiliser des méthodes d'oxydation biomimétique c'est-à-dire associant les molécules donneurs d'oxygène à des métalloporphyrines synthétiques pour modéliser l'action de la ligninase. On peut citer, parmi ces travaux ceux de Shimida et coll utilisant des systèmes catalytiques : alkylhydroperoxyde (ROOH) ferriproto-porphyrine ou méso-tétraphénylporphyrine de fer (III), J. Chem. Soc. Res. Commun., 1323 (1985) et Holzforschnung, 41,277 (1987), de Crawford et coll., même système catalytique que précédemment, Appl. Environ. Microbiol., 54,12 (1988) d'Okamoto et coll utilisant $O_2$/ dihydropyridine / méso-tétraphénylporphyrine de fer (III) comme système catalytique, J. Am. Chem. Soc., 110, 1187 (1988).

Il faut cependant observer que les réactions d'oxydation chimiques mises en oeuvre dans ces processus sont lentes et que les activités catalytiques des systémes décrits sont faibles (de l'ordre de quelques cycles catalytiques par heure).

L'invention vise à pallier ces inconvénients et propose un procédé d'oxydation ménagée de la lignine mettant en oeuvre des systèmes catalytiques présentant une activité élevée.

La présente invention a pour objet un procédé d'oxydation par catalyse biomimétique d'alcools benzyliques et de composés apparentés caractérisé en ce qu'il est réalisé en milieu liquide au moyen d'un catalyseur constitué d'une métalloporphyrine en présence d'un agent oxydant constitué par un sel hydrosoluble d'un peracide organique ou minéral. La porphyrine utilisable dans le procédé de l'invention sera métallée avec le fer ou le manganèse.

Cette porphyrine sera utilisée de préférence sous forme substituée en position méso. Les substituants seront choisis de préférence en série aromatique.

Le peroxyde, sel hydrosoluble d'un peracide, utilisé dans le procédé selon l'invention sera soit minéral, c'est-à-dire se présentant par exemple sous forme d'un sel tel que l'hydrogénopersulfate de potassium, soit organique c'est-à-dire sous la forme d'un sel hydrosoluble d'un peracide tel que l'acide peracétique ou monoperphtalique.

Dans une variante préférée du procédé de l'invention, on utilisera l'hydrogénopersulfate de potassium comme agent oxydant.

Le procédé de l'invention peut être mis en oeuvre soit en catalyse homogène c'est-à-dire avec un système

catalytique en solution dans le milieu réactionnel soit en catalyse supportée, la métalloporphyrine utilisée étant déposée par imprégnation sur un support solide tel que par exemple une résine échangeuse d'ions.

L'utilisation d'une métalloporphyrine supportée sera particuliérement avantageuse dans un procédé industriel dans la mesure où le catalyseur peut être réutilisé après régénération. Celle ci effectuée par lavage et séchage permettra d'obtenir dans le procédé des performances analogues à celles du catalyseur neuf.

Une autre caractéristique importante du procédé de l'invention réside dans le milieu réactionnel utilisé.

Les réactions sont effectuées dans un mélange eau tamponnée-acétonitrile dont la composition influence fortement l'efficacité des réactions catalytiques. Il sera particulièrement avantageux d'utiliser une proportion d'acétonitrile comprise entre 5 et 40% en volume. Au delà de 40% d'acétonitrile, ce dernier risque de jouer le rôle d'inhibiteur de la réaction. On peut observer à ce sujet que lorsque le milieu réactionnel est composé soit d'acétonitrile pur ou d'eau tamponnée seule, l'activité catalytique est pratiquement nulle.

Ce milieu sera acide présentant de préférence un pH de l'ordre de 2 à 3 pour la porphyrine de fer et de l'ordre de 4,5 à 6 pour la porphyrine de manganèse.

Dans une variante du procédé de l'invention, on pourra ajouter au milieu réactionnel un excès d'une base organique azotée telle que la pyridine, l'imidazole et leurs dérivés.

L'addition de cette base organique azotée sera particulièrement avantageuse lorsqu'on utilisera la porphyrine de manganèse que cela soit en catalyse homogène ou en catalyse supportée. Les exemples données ci-après montrent une considérable augmentation d'efficacité de la catalyse par porphyrine de manganèse lorsqu'on ajoute une base pyridinique.

Il faut observer que lorsqu'on utilisera la porphyrine de fer, il ne sera pas utile d'ajouter la base azotée qui n'aura pas d'influence sensible sur la catalyse homogène et il sera même déconseillé de le faire en catalyse supportée, la base azotée ayant un effet inhibiteur dû vraisemblablement à une affinité de la base pour une double fixation sur la porphyrine de fer.

Le procédé de l'invention sera mieux compris à la lecture des exemples ci-après donnés à titre d'illustration.

Les expériences correspondantes ont été réalisées en utilisant comme substrat des molécules alcools benzyliques et composés apparentés qui par ailleurs, sont des modèles de la lignine tels que ceux préconisés par M. Tien et T.K. Kirk (Proc Nat. Acad. Sci USA. Vol 81 pp 2280-2284 - April 1984).

Ces molécules sont ci-après désignées par :

- I pour l'alcool veratrylique et,
- II pour le 1-(3,4-diméthoxyphényl)-2 -(2-méthoxyphénoxy) propane-1,3-diol.

Les expériences ont été réalisées avec comme catalyseur des porphyrines métallées avec le manganèse ou le fer. Ces porphyrines étaient sous la forme M- 5,10,15,20-tétrakis(4-sulfonatophényl)porphyrine tétrasodique ou M-5,10,15,20- tétrakis (3-sulfonato -2,6 dichlorophényl) porphyrine tétrasodique, M étant le métal Mn ou Fe symbolisées respectivement MTPPS et MTDCPS.

Elles étaient soit sous la forme libre, catalyse homogène, soit sous forme supportée après fixation de la porphyrine sur une résine échangeuse d'ions (Amberlite, IRA 900) par imprégnation. Pour préciser que le catalyseur est fixé sur support on adjoindra -Ad à la désignation symbolique.

Préparation des catalyseurs métalloporphyrines

1) MTPPS : dans une première étape, on prépare $TPPSH_2$ 5, 10, 15, 20-tétrakis(4-sulfonatophényl)porphyrine tétrasodique, porphyrine hydrosoluble, obtenue par sulfonation de $TPPH_2$ (5,10,15,20-tétraphénylporphyrine) suivant la méthode de Tsutsui Srivastava et M. Mitsui J. Org.Chem. 38,2103 (1973).

- Analyse : $TPPSH_2$, $8H_2O$, $C_{44}H_{42}N_4O_{20}S_4Na_4$, M = 1166. % C = 45,60 (théor. = 45,20) ; % H = 4,00 (théor. = 3,60).

Dans une deuxième étape, on prépare la MnTPPS : 5, 10, 15, 20-tétrakis(4-sulfonatophényl)porphyrine de manganèse (III) tétrasodique à partir de $TPPSH_2$.

Cinq équivalents d'acétate de manganèse, Mn $(OAc)_2$, $4H_2O$ (210 mg, 8,57 x $10^{-4}$ mole) sont additionnés à une solution de porphyrine $TPPSH_2$ (200 mg, 1,71 x $10^{-4}$ mole) La purification s'effectue comme précédemment. (192 mg de MnTPPS, Rdt = 70 %).

- Analyse : MnTPPS(OAc), 20 $H_2O$ ; $C_{46}H_{27}N_4O_{14}S_4Na_4Mn$, 20 $H_2O$ M = 1494. % C = 38,4 (théor. = 38,30) ; % H = 3,92 (théor. = 4,65), % N = 3,72 (théor. = 3,89).

La préparation de FeTPPS : 5,10,15,20-tétrakis (4-sulfonatophényl) porphyrine de fer (III) tétrasodique s'effectue selon une procédure analogue.

Cinq équivalents de sel de Mohr, $(NH_4)_2Fe(SO_4)_2$, $6H_2O$ (333 mg, 8,5 x $10^{-4}$ mole) sont additionnés à une solution de porphyrine (200 mg, 1,71 x $10^{-4}$ mole) dans 30 ml d'eau. Le pH de la solution doit être compris entre 5 et 8 pour une meilleure métallation. Le reflux est maintenu pendant une heure (200 mg, Rdt = 75 %).

- Analyse : FeTPPS, $(NH_4)_2SO_4$ $_2$12 $H_2O$ ; $C_{44}H_{64}N_8O_{32}S_6Na_4Fe$, M = 1556. % C 33,70 (théor. = 33,93) ; % H = 4,14 (théor. = 4,11), % N = 6,53 (théor. = 7,20).

Préparation du catalyseur supporté MnTPPS-Ad par immobilisation du MnTTPS sur résine échangeuse d'ions Amberlite IRA 900.

L'immobilisation est réalisée suivant la méthode de Y. Saito et coll. (Pure and Appl. Chem., 59,573 (1987)) : addition de 2 g de résine échangeuse d'ions Amberlite IRA 900 (produit Aldrich), préalablement lavée à l'eau puis au méthanol et au mélange acétone-eau (1/1, v/v) à une solution de MnTPPS (100 mg, 5 x $10^{-4}$ M) dissoute dans 200 ml d'un mélange acétone-eau (1/1, v/v). L'immobilisation est réalisée à 35 - 40°C pendant

30 minutes, jusqu'à l'éclaircissement optimal de la solution. La résine récupérée est lavée avec un mélange acétone-eau (1/1, v/v) puis séchée (5 mg de métalloporphyrine MnTPPS sont fixés sur 100 mg d'Amberlite). Le décrochage de la métalloporphyrine est impossible, tant en milieu acide qu'en milieu basique.

La préparation de FeTPPS-Ad est réalisée dans les mêmes conditions que pour MnTPPS-Ad. Le temps nécessaire à l'immobilisation à 40°C est de 4 heures.

2) MTDCPS : dans une première étape on prépare $H_2TDCP$ suivant la méthode décrite par A.W. van der Made et al. Rec. Trav. Chim. Pays Bas 107, 15-16 (1988) puis dans une deuxième étape on prépare $H_2TDCPS$ par sulfonation de $H_2TDCP$ au moyen d'oléum à 120°C pendant 4 à 5 heures. Le rendement obtenu est de 85 %.

Les étapes ultérieures de métallation par le manganèse ou le fer et le cas échéant l'imprégnation sur le support (Amberlite) sont analogues à celles décrites ci-dessus pour MTPPS.

<u>Conditions générales des essais catalytiques.</u>

La conversion des substrats I ou II transformés lors de la réaction catalytique est mesurée par analyse HPLC.

La solution oxydante utilisée est, sauf indication contraire, pour les exemples comparatifs, à base d'hydrogénopersulfate de potassium $KHSO_5$.

- Dans le cas de la catalyse homogène utilisant le MTPPS libre (M = Fe, Mn), le substrat, 20 μmol dans 500 μl d'acétonitrile et le catalyseur MTPPS, 2 μmol dans 1 ml de tampon citrate - phosphate 0,1 M ; pH = 3,0, sont préalablement mélangés. L'addition de la solution oxydante, 100 μmol d'Oxone R : $2KHSO_5$, $KHSO_4$, $K_2SO_4$, 30,7 mg déclenche la réaction catalytique menée à la température ambiante.

- Dans le cas de la catalyse supportée utilisant le MTPPS-Ad (M = Fe, Mn) le catalyseur MTPPS-Ad (100 mg) est ajouté à la solution de substrat, 20 μmol dans 1,5 ml de solution acétonitrile-tampon, pH = 3,0 (1/1, v/v). La réaction catalytique est également déclenchée par l'addition de la solution oxydante.

L'addition de la base azotée, pyridine et 4-tert-butylpyridine, en excès, 150 équivalents par rapport au substrat, est effectuée lors de la dissolution du substrat dans l'acétonitrile. Des prélèvements sont effectués toutes les minutes pendant les cinq premières minutes, puis toutes les cinq minutes jusqu'à 30 minutes puis analysés en HPLC.

Dans toutes ces réactions on met en jeu un milieu réactionnel constitué de 25 % d'acétonitrile et de 75 % de tampon citrate-phosphate 0,1 M, pH = 3,0 (en volume).

Les conditions expérimentales des exemples 1 à 23 sont les suivantes :

Exemple 1 :

Substrat I, (20 μmol ; 3,36 mg), FeTPPS-Ad (100 mg ; tampon citrate - phosphate 0,1 M pH = 3,0 (1,5 ml) ; $H_2O_2$ (10 μmol) 24,7 μl d'une solution aqueuse à 30 %, de molarité 4,05).

Exemple 2 :

Mêmes conditions expérimentales que l'exemple 1. Remplacement de $H_2O_2$ par $KHSO_5$ (100 μmol ; 30,7 mg).

Exemple 3 :

Substrat II (20 μmol, 6,68 mg), FeTPPS libre (2 μmol ; 3,11 mg) ; $CH_3CN$ (500 μl), tampon citrate-phosphate 0,1 M, pH = 3,0 (1,5 ml), $H_2O_2$ (10 μmol ; 24,7 μl).

Exemple 4 :

Mêmes conditions que pour l'exemple 3. Remplacement de $H_2O_2$ par $KHSO_5$ (100 μmol ; 30,7 mg).

Exemple 5 :

Substrat I (20 μmol ; 3,36 mg) ; MnTPPS libre (2 μmol ; 2,98 mg) ; $CH_3CN$ (500 μl) ; tampon citrate-phosphate 0,1 M, pH = 3,0 (1,5 ml) ; $KHSO_5$ (100 μmol ; 30,7 mg).

Exemple 6 :

Mêmes conditions expérimentales que pour l'exemple 5 à l'exception du pH égal à 6. Addition de 4-tert-butylpyridine (300 μmol ; 43 μl).

Exemple 7 :

Substrat II (20 μmol ; 6,68 mg) MnTPPS Libre (2 μmol ; 2,98 mg) ; $CH_3CN$ (500 μl) ; tampon pH = 3,0 (1,5 ml) 4-tert-butylpyridine (300 μmol ; 43 μl) ; $KHSO_5$ (100 μmol ; 30,7 mg).

Exemple 8 :

Mêmes conditions expérimentales que pour l'exemple 5. Catalyseur : MnTPPS-Ad (100 mg).

Exemple 9 :

Substrat II (20 μmol ; 6,68 mg) ; MnTPPS-Ad (100 mg ) ; $CH_3CN$ (500 μl) ; tampon pH = 3,0 (1,5 ml) ; $KHSO_5$ (100 μmol ; 30,7 mg).

Exemple 10 :

Mêmes conditions expérimentales que pour l'exemple 8. Addition de pyridine (300 µmol ; 29 µl).

Exemple 11 :

Mêmes conditions expérimentales que pour l'exemple 8. Addition de 4-tert-butylpyridine (300 µmol ; 43 µl) ; et pH fixé à 6.

Exemple 12 :

Mêmes conditions expérimentales que pour l'exemple 11 le pH étant fixé à 3. Substrat II (20 µmol ; 6,68 mg).

Exemple 13 :

Substrat I (20 µmol ; 3,36 mg) ; FeTPPS libre (0,2 µmol ; 3,11 mg) ; $CH_3CN$ (500 µl) ; tampon citrate-phosphate 0,1 M, pH = 3,0 (1,5 ml) ; $KHSO_5$ (100 µmol ; 30,7 mg).

Exemple 14 :

Mêmes conditions expérimentales que pour l'exemple 13. Substrat II (20 µmol ; 6,68 mg).

Exemple 15 :

Mêmes conditions expérimentales que pour l'exemple 13. Catalyseur : FeTPPS-Ad (100 mg).

Exemple 16 :

Mêmes conditions expérimentales que pour l'exemple 15. Addition de 4-tert-butylpyridine (300 µmol ; 43 µl).

Exemple 17 :

Mêmes conditions expérimentales que pour l'exemple 15, avec substrat II (20 µmol ; 6,68 mg).

Exemple 18 :

Mêmes conditions expérimentales que pour l'exemple 17. Addition de 4-tert-butylpyridine (300 µmol ; 43 µl).

Exemple 19 :

Mêmes conditions expérimentales que pour l'exemple 13 en remplaçant $KHSO_5$ par le sel de magnésium de l'acide monoperphtalique.

Exemple 20 :

Substrat I, 120 µmol, 500 µl d'une solution d'acitonitrile à 40 mmol en alcool veratrylique ; Fe TDCPS (200 nmol; 100 µl d'une solution de porphyrine à 2 mmol tamponnée à pH = 3,0 ; tampon citrate-phosphate 0,1 M (900 µl) ; $KHSO_5$ (100 µmol ; 30,7 mg dissous dans 500 µl de tampon pH = 3,0)
La concentration en porphyrine est 10 fois plus faible que dans les exemples précédents.

Exemple 21 :

Substrat I (20 µmol ; 500 µl d'une solution d'acétonitrile à 40 mM en alcool veratrylique) ; Mn TDCPS (2 µmol ; 3,2 mg dissous dans 500 µl de tampon 0,5 M pH = 6,0 ; 4 tert-butylpyridine (200 µmol ; 28 µl) $KHSO_5$ (100 µmol ; 30,7 mg dissous dans 500 µl de tampon phosphate 0,5 M pH = 6.

Exemple 22 :

Mêmes conditions opératoires que pour l'exemple 20 en remplaçant le substrat I par le substrat II.

Exemple 23 :

Mêmes conditions opératoires que pour l'exemple 21 en remplaçant le substrat I par le substrat II.

Les produits formés lors de l'oxydation catalytique de l'alcool vératrylique sont le 3,4-diméthoxybenzaldéhyde et la 2-méthoxy-5- hydroxyméthyl - 1,4 - benzoquinone. Dans le cas de l'oxydation du substrat II, on obtient parmi les produits d'oxydation le 3,4 diméthoxybenzaldéhyde et la 2-méthoxy- 1-4 benzoquinone.

Les résultats des exemples 1 à 23 sont donnés dans le tableau 1 ci-après.

TABLEAU 1

| Exemple | Catalyseurs | Substrat | L(a) | Donneur d'oxygène (b) | %Conversion après(mn) | | | |
|---|---|---|---|---|---|---|---|---|
| | | | | | 1 | 2 | 5 | 10 |
| 1 | FeTPPS-Ad | I | | $H_2O_2$ | - | 1 | 2 | 4 |
| 2 | idem | I | | $KHSO_5$ | 28 | 52 | 60 | 60 |
| 3 | FeTPPS | II | | $H_2O_2$ | 22 | - | | 26 |
| 4 | idem | II | | $KHSO_5$ | 100 | | | - |
| 5 | MnTPPS | I | | idem | 0 | 0 | 0 | 0 |
| 6 | idem | I | $L_2$ | " | 100 | | | |
| 7 | idem | II | $L_2$ | " | 100 | | - | |
| 8 | MnTPPS-Ad | I | | " | 6 | 11 | 14 | 14 |
| 9 | idem | II | | " | 25 | | 34 | 35 |
| 10 | idem | I | $L_1$ | " | 34 | | 36 | 36 |
| 11 | idem | I | $L_2$ | " | 90 | 100 | | |
| 12 | idem | II | $L_2$ | " | 100 | | | |
| 13 | FeTPPS | I | | " | 100 | | | |
| 14 | idem | II | | " | 100 | | | |
| 15 | FeTPPS-Ad | I | | " | 28 | 52 | 60 | 60 |
| 16 | idem | I | $L_2$ | " | 26 | 28 | 30 | |
| 17 | idem | II | | " | 55 | 63 | 63 | |
| 18 | idem | II | $L_2$ | " | 3 | 6 | 8 | 10 |
| 19 | FeTPPS | I | | MMPP | 100 | | | |
| 20 | FeTDCPS | I | | $KHSO_5$ | 100 | | | |
| 21 | MnTDCPS | I | $L_2$ | " | 70 | | | 85 |
| 22 | FeTDCPS | II | | " | 50 | | | 63 |
| 23 | MnTDCPS | II | $L_2$ | " | 100 | | | |

a) L désigne la base azotée, $L_1$ = pyridine ; $L_2$ = 4-tert-butylpyridine.
b)MMPP désigne le sel de magnésium de l'acide monoperphtalique.

Les exemples 1 à 4 illustrent l'efficacité obtenue par l'utilisation dans le procédé de l'invention d'un sel de peracide comme agent donneur d'oxygène en comparaison avec l'eau oxygénée.

Deux donneurs d'atome d'oxygène, l'eau oxygénée $H_2O_2$ (solution aqueuse à 30 %) et le monopersulfate de potassium, $KHSO_5$, ont été utilisés et comparés. Le complexe FeTPPS-Ad associé à l'eau oxygénée, exemple 1, ne donne que 4 à 5 % de conversion du substrat I. Le monopersulfate de potassium permet d'obtenir 60 % de conversion de I en 5 minutes (exemple 2). Cet effet est également observé pour le substrat II avec la porphyrine de fer libre (exemples 3 et 4).

Les exemples 5 à 12 illustrent l'utilisation de la porphyrine de manganèse comme catalyseur de l'oxydation des substrats I (exemples 5 et 6) et II (exemple 7) en catalyse homogène et des substrats I (exemples 8, 10 et 11) et II (exemples 9 et 12) en catalyse supportée.

Tous les essais mettent en jeu 20 µmol de substrat, 2 µmol de catalyseur (soit 10 % par rapport au substrat) et 100 µmol d'hydrogénopersulfate de potassium (soit 5 équivalents par rapport au substrat). Pour les deux substrats, la métalloporphyrine libre (catalyse homogène) en l'absence de base azotée (pyridine ou 4-tert-butylpyridine par exemple) n'a pas d'activité catalytique notable. Par contre, lorsque la porphyrine de manganèse est adsorbée sur la résine, on peut noter une activité pour les deux substrats (exemples 8 et 9). Pour le substrat I cette activité est fortement augmentée en présence de 150 équivalents par rapport au substrat de pyridine ou de 4-tert-butylpyridine qui jouent alors le rôle de ligand proximal.

On peut observer que la 4-tert-butylpyridine donne de meilleurs résultats que la pyridine elle-même (exemples 10 et 11). Cet effet se retrouve également dans le cas de la porphyrine de manganèse libre avec la 4-tert-butylpyridine (exemple 6).

Pour le substrat II, on observe la même influence favorable de la 4-tert-butylpyridine, puisque la conversion de II est totale en moins d'une minute (exemple 12). La même activité est obtenue dans le cas de la porphyrine de manganèse libre avec la même base azotée (exemple 7). Ce résultat correspond à une activité catalytique supérieure à 10 cycles catalytiques par minute, très supérieure à celles décrites précédemment dans la littérature pour d'autres systèmes d'oxydation. Dans le cas du système $Fe^{III}$ (porphyrine)/ROOH, l'activité est de 1 cycle / heure et pour $Fe^{III}$ (porphyrine)/$O_2$/réducteur, celle-ci ne dépasse pas non plus 1 cycle/heure. (cf M. Shimida et coll. Holzforschung 41,277(1987).

Par ailleurs, le catalyseur supporté est parfaitement recyclable puisque, récupéré lors de l'essai 11 (72 % de

conversion de I), lavé et séché, il a permis d'obtenir, dans les mêmes conditions que l'essai 11, 60 % de conversion de I.

Les exemples 13 à 19 illustrent l'utilisation de la porphyrine de fer comme catalyseur du procédé.

Contrairement au cas du manganèse, la porphyrine de fer libre présente une très forte activité catalytique : la conversion du substrat I ou du substrat II est totale en moins d'une minute (exemples 13 et 14) pour des quantités de catalyseur représentant moins de 1% (molaire) du substrat.

Dans le cas des porphyrines de fer, il est connu que contrairement aux phorphyrines de manganèse la constante d'affinité pour la fixation d'une deuxième base azotée en position axiale est plus grande que celle correspondant à la fixation de la première molécule.

Dans ces conditions, la présence de la 4-tert-butylpyridine devrait inhiber la conversion des substrats I et II. C'est le cas pour le catalyseur adsorbé sur résine (exemples 16 et 18, pour les substrats I et II). Dans le cas du substrat II, on passe d'une conversion de 67 % pour FeTPPS-Ad (exemple n° 17) à 7 % pour FeTPPS-Ad ($L_2$) (exemple 18), pour un temps de réaction de 5 minutes. Cet effet d'inhibition n'est pas observé pour la porphyrine de fer libre. En présence de 4-tert-butylpyridine, la conversion de I et II est toujours complète en une minute de réaction, ce qui indique que l'activité est bien au-delà de 10 cycles par minute dans le cas des essais sans pyridine.

La réaction d'oxydation est effectuée dans un mélange eau tamponnée /acétonitrile. Il a été observé que la composition du mélange influence fortement l'efficacité des réactions catalytiques.

Dans l'exemple 24 on a mesuré la conversion du substrat I par oxydation au moyen de FeTPPS-Ad en présence de $KHSO_5$ à une minute et deux minutes de réaction en faisant varier le pourcentage d'acétonitrile dans le milieu réactionnel.

Les résultats obtenus sont donnés dans le tableau 2.

TABLEAU 2

| % Acétonitrile (vol) | 0 | 10 | 20 | 25 | 30 | 60 | 100 |
|---|---|---|---|---|---|---|---|
| Taux conversion (1 mn) | 0 | 22 | 29 | 30 | 29 | 8 | 0 |
| Taux conversion (2 mn) | 0 | 34 | 40 | 42 | 40 | 22 | 0 |

Les pourcentages de conversion, mesurés à une minute ou deux minutes de réaction, augmentent lorsque la proportion d'acétonitrile augmente jusqu'à 25-30 % en volume. Par contre, la conversion est fortement diminuée lorsque cette proportion continue d'augmenter. A partir de 40 % d'acétonitrile, on peut considérer que celui-ci joue le rôle d'ion inhibiteur de la réaction. Lorsque le milieu réactionnel est composé d'acétonitrile pur ou de tampon seul, l'activité catalytique est pratiquement nulle.

L'exemple 25 illustre des essais comparatifs visant une optimisation des systèmes catalytiques à base de porphyrines de manganèse. Ces essais ont été réalisés au moyen du substrat I et du système MnTPPS/$KHSO_5$.

L'étude a été réalisée dans des conditions générales d'essais catalytiques analogues à celles des exemples 5 et 6 pour MnTPPS , en étudiant, d'une part la diminution de la proportion catalyseur/substrat (%) et d'autre part l'influence de la 4-tert-butylpyridine ($L_2$). Les résultats obtenus sont représentés dans le tableau 3.

L'étude du système catalytique MnTPPS/$KHSO_5$ (tableau 3) montre que pour un pourcentage en catalyseur inférieur à 5%, on obtient une faible conversion de I. Par contre l'influence d'un ligand axial basique (de type pyridine) est déterminante sur l'activité catalytique du système. Dans le cas des essais à 10 % en catalyseur par rapport au substrat, la conversion de I est plus importante lorsqu'on augmente le nombre d'équivalents de $L_2$ par rapport au catalyseur. A 5 % en catalyseur avec 150 équivalents de $L_2$ par rapport au catalyseur, on obtient une activité catalytique égale à 1 cycle par seconde.

TABLEAU 3

| Catalyseur (%/Substrat) | Ligand axial $L_2$ (nbre d'équiv/catalyseur) | Conversion de de I en 1 mn % |
|---|---|---|
| 10 | 20 | 3 |
| idem | 100 | 48 |
| idem | 150 | 58 |
| 5 | 20 | 9 |
| idem | 100 | 30 |
| idem | 150 | 45 |
| idem | 300 | 66 |
| 0,2 | 0 | 0 |
| idem | 100 | 5 |
| 0,1 | 0 | 0 |
| idem | 100 | <2 |

Exemple 26 :

Afin de tester l'efficacité de ces catalyseurs modèles de la ligninase, un test de délignification a été réalisé sur de la pâte de feuillu lyophilisée. La détermination du degré de délignification est réalisée par la mesure de l'indice kappa (norme AFNOR, NF T12-018-1980).

La pâte de feuillu a été lyophilisée avant traitement ; sa teneur en eau était de 1,8 % et l'indice kappa était de 12,5. 1g de pâte lyophylisée, soit environ 200 µmol de composés aromatiques, est mis en suspension dans 100 ml de tampon citrate-phosphate 0,1 M pH = 3,0. On ajoute ensuite le catalyseur FeTPPS : 12 µmol ; 18 mg dans 10 ml de tampon et le milieu est agité pendant 2 heures afin de fixer partiellement le catalyseur sur la pâte. La réaction est ensuite déclenchée par addition d'une solution de $KHSO_5$ : 1,4 mmol ; 450 mg dans 10 ml de tampon pH = 3,0. Après 12 heures de réaction la pâte est filtrée, lavée à l'eau puis à l'acétone. L'indice kappa mesuré sur l'échantillon récupéré et lyophilisé est égal à 7,2.

**Revendications**

1 - Procédé d'oxydation par catalyse biomimétique d'alcools benzyliques et de composés apparentés caractérisé en ce qu'il est réalisé en milieu liquide en présence d'un catalyseur constitué d'une métalloporphyrine et d'un dérivé hydrosoluble d'un peracide minéral ou organique.

2 - Procédé selon la revendication 1 caractérisé en ce que la métalloporphyrine est une porphyrine de manganèse.

3 - Procédé selon la revendication 2 caractérisé en ce que le dérivé du peracide est le monopersulfate de potassium.

4 - Procédé selon la revendication 3 caractérisé en ce que la porphyrine de manganèse est déposée par imprégnation sur un support solide.

5 - Procédé selon la renvendication 4, caractérisé en ce que le support solide est une résine échangeuse d'ions.

6 - Procédé selon la revendication 2 caractérisé en ce que le catalyseur est mis en oeuvre dans un milieu réactionnel comprenant de 5 à 40 % en volume d'acétonitrile.

7 - Procédé selon la revendication 4 caractérisé en ce que la réaction est réalisée en présence d'un excès d'une base organique azotée.

8 - Procédé selon la revendication 3 caractérisé en ce que la porphyrine de manganèse est mise en oeuvre en solution dans le milieu réactionnel.

9 - Procédé selon la revendication 8 caractérisé en ce que le milieu réactionnel comprend de 5 à 40 % en volume d'acétonitrile.

10 - Procédé selon la revendication 9 caractérisé en ce que la réaction est réalisée en présence d'un excès d'une base organique azotée.

11 - Procédé selon la revendication 1 caractérisé en ce que la métalloporphyrine est une porphyrine de fer.

12 - Procédé selon la revendication 11 caractérisé en ce que le dérivé du peracide est le monopersulfate de potassium.

13 - Procédé selon la revendication 12, caractérisé en ce que la porphyrine de fer est mise en oeuvre en solution dans le milieu réactionnel.

14 - Procédé selon la revendication 13 caractérisé en ce que le catalyseur est mis en oeuvre dans un

milieu réactionnel comprenant de 5 à 40 % en volume d'acétonitrile.

15 - Procédé selon la revendication 12 caractérisé en ce que la porphyrine de fer est déposée par imprégnation sur un support solide.

16 - Procédé selon la revendication 15 caractérisé en ce que le support solide est une résine échangeuse d'ions.

17 - Procédé selon la renvendication 15 caractérisé en ce que le catalyseur est mis en oeuvre dans un milieu réactionnel comprenant 5 à 40 % en volume d'acétonitrile.

Office européen des brevets

# RAPPORT DE RECHERCHE EUROPEENNE

Numero de la demande

EP 89 40 1920

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | BIOCHEMICAL AND BIOPHYSICAL RESEARCH COMMUNICATIONS, vol. 122, no. 3, 16 août 1984, pages 1247-1252, Academic Press, Inc.; M. SHIMADA et al.: "The C-C bond cleavage of a lignin model compound, 1,2-diarylpropane-1,3-diol, with a heme-enzyme model catalyst tetraphenylporphyrinatoiron(III)chloride in the presence of tert-butylhydroperoxide" * Pages 1248-1250 * --- | 1,11,13 | C 07 C 45/29<br>C 07 C 47/575<br>C 07 C 46/02<br>C 07 C 50/28 |
| D,Y | J. AM. CHEM. SOC., vol. 110, 1988, pages 1187-1196, American Chemical Society; T. OKAMOTO et al.: "Biomimetic oxidation with molecular oxygen. Selective carbon-carbon bond cleavage of 1,2-diols by molecular oxygen and dihydropyridine in the presence of iron-porphyrin catalysts" * Pages 1188-1190,1195 * --- | 1,11,13 | **DOMAINES TECHNIQUES RECHERCHES (Int. Cl.5)** |
| Y | J. CHEM. SOC., CHEM. COMMUN., 1985, pages 381-382; T. OKAMOTO et al.: "Catalysis of aerobic C-C bond cleavage of 1,2Bis(4-methoxyphenyl)ethane-1,2-diol by meso-tetraphenylporphyrinatoiron(III). A model system for cytochrome P-450scc dependent glycol cleavage" * Pages 381-382 * ----- | 1,11,13 | C 07 C 45/00<br>C 07 C 47/00<br>C 07 C 46/00<br>C 07 C 50/00 |

Le présent rapport a été établi pour toutes les revendications

| Lieu de la recherche | Date d'achèvement de la recherche | Examinateur |
|---|---|---|
| LA HAYE | 09-10-1989 | BONNEVALLE E.I.H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)